# EUROPEAN PATENT SPECIFICATION

(11) **EP 2 373 612 B1**
(45) Date of publication and mention of the grant of the patent: **17.04.2013**
(21) Application number: 09796625.3
(22) Date of filing: 16.12.2009
(51) Int. Cl.: C07C 209/62, C07C 211/38

(54) **METHOD FOR PRODUCING MEMANTINE**
VERFAHREN ZUR HERSTELLUNG VON MEMANTIN
PROCÉDÉ DE PRODUCTION DE MÉMANTINE

(30) Priority: 17.12.2008 EP 08021951; 17.12.2008 US 201986 P
(43) Date of publication of application: 12.10.2011
(73) Proprietor: Merz Pharma GmbH & Co. KGaA, 60318 Frankfurt am Main (DE)
(72) Inventor: GOLD, Markus-Rene, 97753 Karlstadt (DE); JIRGENSONS, Aigars, LV-1013 Riga (LV); HUBER, Florian, Anton, Martin, 30031 Dolo (VE) (IT)
(74) Representative: Ricker, Mathias
(86) International application number: PCT/EP2009/009029
(87) International publication number: WO 2010/069555

(56) References cited:
- WO-A-2006/122238
- WO-A-2007/101536
- GB-A- 925 728
- DATABASE CA [Online] CHEMICAL ABSTRACTS SERVICE, COLUMBUS, OHIO, US; 2007, LAVROVA, L. N. ET AL: "Process for preparation of 3,5-dimethyl-1-adamantylamine or its salts by sequential bromination, formamidation and acid hydrolysis steps" XP002528843 retrieved from STN Database accession no. 2007:1283216 cited in the application & RU 2 309 940 C2 (NPTS "FARMZASHCHITA", RUSSIA) 10 November 2007 (2007-11-10)
- REDDY, JAMBULA MUKUNDA ET AL: "An Improved Synthesis of Memantine Hydrochloride: Anti-Alzheimer's Drug" ORGANIC PROCESS RESEARCH & DEVELOPMENT , 11(2), 268-269 CODEN: OPRDFK; ISSN: 1083-6160, 2007, XP002528841 cited in the application
- GERZON, K: "The Adamantyl Group in Medicinal Agents. Hypoglycemic N-Arylsulfonyl-N'-adamantylureas" J. MED. CHEM, vol. 6, 1963, pages 760-763, XP002528842
- STETTER H ET AL: "Compounds with urotropine structure. XVI. The chemistry of 1-adamantyl derivatives . Ueber Verbindungen mit Urotropin-Struktur, XVI. Beitraege zur Chemie der Adamantyl-(1)-Derivate", CHEMISCHE BERICHTE, VERLAG CHEMIE GMBH. WEINHEIM, DE, no. 93, 1 January 1960 (1960-01-01), pages 226-230, XP002439717, ISSN: 0009-2940, DOI: 10.1002/CBER.19600930133

## Description

### FIELD OF THE INVENTION

The invention relates to a method for producing 1-amino-3,5-dimethyladamantane or a salt thereof. Specifically, the invention relates to a method for producing 1-amino-3,5-dimethyladamantane or a salt thereof by heating 1-formamido-3,5-dimethyl-adamantane with a base in a protic solvent. The invention also relates to the conversion of 1-amino-3,5-dimethyladamantane to a pharmaceutically acceptable salt thereof.

### BACKGROUND OF THE INVENTION

The hydrochloride salt of 1-amino-3,5-dimethyladamantane is an important active ingredient and is also known under the name "Memantine". Memantine is sold in Europe and in numerous non-European countries under the trademarks Axura® and Ebixa® and in the USA under the trademark Namenda®. Memantine is an effective medicament against, among others, Alzheimer's disease.

DE 1 197 091 discloses the hydrolysis of 1-formamidoadamantane with sodium hydroxide in refluxing diethylene glycol (boiling point: 245°C) to 1-aminoadamantane in a yield of 70 %. The hydrolysis time leading to the unsubstituted aminoadamantane is 10 h.

In the processes known from the art, Memantine (i.e. a substituted 1-formamidoadamantane) is typically obtained by hydrolysis of either N-acetamido or N-formamido intermediates.

From BE 646,581 it is known to prepare 1-aminoadamantane by heating 1-acetamidoadamantane with sodium hydroxide in diethylene glycol at a temperature of 180 °C and at a hydrolysis time of 5 h. No yield is disclosed.

WO 2006/076562 A1 discloses the alkaline hydrolysis of 1-acetamido-3,5-dimethyladamantane to 1-amino-3,5-dimethyladamantane requiring very high reaction temperatures, typically from 190 °C to 225 °C.

The basic hydrolysis of N-acetamido compounds requires harsh reaction conditions, i.e. high temperatures (>180°C), strong alkaline conditions and relatively long hydrolysis times.

In another process for producing Memantine, WO 2007/101536 A1 suggests to hydrolyze 1-formamido-3,5-dimethyladamantane to 1-amino-3,5-dimethyladamantane under acidic conditions. Aqueous hydrochloric acid is used as acid, wherein the starting material and acid needs to be refluxed for 24 hours in order to obtain an acceptable yield of final product. Memantine is isolated in the form of the corresponding hydrochloride salt in a yield of approximately 80 %.

WO 2006/122238 relates to a low yield process for preparing Memantine or an acid addition salt of Memantine adamantane based on reacting 1-bromo-3,5-dimethyl adamantane with formamide to form 1-formamido-3,5-dimethyl adamantane. The process involves acid hydrolysis of the intermediate.

A Russian article by L.N. Lavrova et al. (XP-002528843) with the title "Process for preparation of 3,5-dimethyl-1-adamantylamine or its salts by sequential bromination, formidation and acid hydrolysis steps*"* discloses acid hydrolysis of a formyl derivative.

An article by J.M. Reddy et al. (XP-002528841) with the title "An Improved Synthesis of Memantine Hydrochloride: Anti-Alzheimers Drug*"* relates to a process involving the conversion of 1,3-dimethyl adamantine to a formamide intermediate as a key step, followed by acid hydrolysis to (1-amino-3,5-dimethyl adamantane) hydrochloride.

On the industrial scale, acid hydrolysis has the drawback of an extended hydrolysis time that causes increased production costs due to an unfavorable space-time-yield. Furthermore, said acidic conditions using hydrochloric acid may lead to unwanted by-products

Most importantly, acid hydrolysis of formamido adamantanes typically employs aqueous hydrochloric acid. This is convenient for the preparation of the hydrochloride salt form (i.e. Memantine hydrochloride), but not for other pharmaceutically acceptable organic and inorganic salts. In case these other salts are desirable, the hydrochloride salt of amino-3-5-dimethyl adamantane subsequently needs to be treated with base to obtain the free base of the amine. Such free base then can be reacted with the corresponding organic and inorganic salts to form the desired pharmaceutically acceptable salt. Overall, three steps are necessary to reach the final product starting from the formamido adamantane and using acid hydrolysis.

GB 925 728 relates to a process for the production of N-tertiary alkyl amides and N-tertiary alkyl amines. Example 3 of this reference discloses the hydrolysis of N-adamantyl-1-formamide using a refluxing solution of sodium hydroxide in diethylene glycol.

Gerzon K. et al., "The Adamantyl Group in Medicinal Agents. I. Hypoglycemic N-Arylsulfonyl-N'-adamantylureas", J. Med. Chem. Vol. 6, 1963, pages 760 - 763, disclose the deacetylation of 1-acetamido-3-methyl-adamantane using potassium hydroxide in diethylene glycol.

Stetter H. et al., "Über Verbindungen mit Urotropinstruktur, XVI, Beiträge zur Chemie der Adamantyl-(1)-Derivate", Chem. Ber. 93, Seiten 226 - 230 (1960), disclose the alkaline hydrolysis of 1-acetamido-adamantane using sodium hydroxide in refluxing diethylene glycol.

### OBJECTS OF THE INVENTION

It was an object of the invention to improve the space-time-yield of the hydrolysis reaction of 1-formamido-3,5-dimethyladamantane to 1-amino-3,5-dimethyladamantane, i.e. decreasing the hydrolysis time while minimizing by-products and maintaining a good product yield.

### SUMMARY OF THE INVENTION

It has been surprisingly found in the present invention that the space-time-yield of the hydrolysis reaction of 1-formamido-3,5-dimethyl-adamantane to 1-amino-3,5-dimethyl-adamantane may be considerably improved if the hydrolysis reaction is carried out under basic conditions in a protic solvent. Hydrolysis times of approximately only 1 hour are achievable, wherein said hydrolysis reaction results in a high conversion degree of the formamido compound to the corresponding amino compound. It was further surprisingly found that comparatively mild conditions, i.e. low hydrolysis temperatures are sufficient and lead to high yields of the free base of 1-amino-3,5-dimethyladamantane.

Memantine in the form of the hydrochloride may be obtained in yields that are comparable to the yield of the processes of the prior art. Surprisingly, no discernible formation of undesired 1-hydroxy-3,5-dimethyl-adamantane was observed that could be expected due to a possible side-reaction, i.e. the substitution of the formamido group by a hydroxyl group. Also no rearrangement reactions were observed that may occur in the 3,5-dimethyladamant-1-yl system resulting in products having the methyl groups in other positions than the 3- and/or 5-position.

Treatment of the free base of 1-amino-3,5-dimethyladamantane with aqueous or gaseous hydrochloric acid gives Memantine in the form of the hydrochloride salt with a high purity, well defined PSD.

Furthermore the free base of 1-amino-3,5-dimethyladamantane is conveniently obtained in a single step. It can be reacted with a wide range of organic and inorganic acids to form other desired pharmaceutically acceptable salts directly, thus avoiding the longer sequence of acid hydrolysis followed by base liberation.

Accordingly, the invention relates to a method for producing 1-amino-3,5-dimethyladamantane or a salt thereof as defined in claim 1.

The base is selected from hydroxides and alkoxides and oxides, carbonates and hydrogen carbonates of alkali metals and earth alkali metals.

The protic solvent is selected from alcohols having from 1 to 8 carbon atoms; ethylene glycol, oligo(ethylene glycol).

The protic solvent comprises from 10 to 60 % by weight water based on the total amount of water and solvent used in the hydrolysis reaction according to step (i).

In one embodiment, the solvent is methanol that comprises from 20 to 50 % by weight water based on the total amount of water and solvent, and the base is sodium hydroxide or potassium hydroxide.

In one embodiment, step (i) is carried out at a temperature of from 60 to 200 °C.

In one embodiment, the molar ratio of 1-formamido-3,5-dimethyl-adamantane to base is from 1:1 to 1:6.

In one embodiment, the amount of the protic solvent is from 10 to 50 % by weight based on the total amount of 1-formamido-3,5-dimethyladamantane, base and protic solvent.

In one embodiment, the method further comprises:
(ii) separating 1-amino-3,5-dimethyladamantane obtained in step (i) from the base and the protic solvent.

In a further embodiment, the method further comprises:
(iii) adding an acid to 1-amino-3,5-dimethyladamantane obtained in step (i) or in step (ii).

In one embodiment, the acid is hydrochloric acid.

In one embodiment, step (ii) further comprises step (ii'):
(ii') dissolving 1-amino-3,5-dimethyladamantane in an aprotic solvent.

In one embodiment, said aprotic solvent is an alkane.

In one embodiment, said alkane is selected from the group consisting of pentane, hexane, heptane, octane, nonane, decane, or a mixture of two or more of said solvents.

In one embodiment, said alkane is heptane.

In one embodiment, said method further comprises step (iv):
(iv) subjecting said aprotic solvent comprising said dissolved 1-amino-3,5-dimethyladamantane obtained in step (ii') to a purification step.

In one embodiment, said purification step is effected by means of charcoal.

In one embodiment, said method further comprises step (v):
(v) adding hydrogen chloride to said aprotic solvent comprising said dissolved 1-amino-3,5-dimethyladamantane obtained in step (ii') or step (iv).

In one embodiment, said method further comprises step (vi):
(vi) heating the mixture obtained in step (v) to a temperature of from 50 to 75 °C.

### DETAILED DESCRIPTION OF THE INVENTION

The present invention may be understood more readily by reference to the following detailed description and to embodiments of the invention and the examples included therein.

The invention relates to a method for producing 1-amino-3,5-dimethyladamantane or a salt thereof as defined in claim 1.

In publications, frequently names such as "1-formylamido-3,5-dimethyladamantane", "1-formylamino-3,5-dimethyladamantane" or "N-(3,5-dimethyladamant-1-yl)-formamide" are synonymously used for 1-formamido-3,5-dimethyladamantane.

Said 1-formamido-3,5-dimethyladamantane is a known product or may be produced according to known methods. For example, said compound may be produced from 1,3-dimethyladamantane under acidic conditions by reaction with formamide.

Such a synthesis is e.g. known from WO 2007/101 536 A1. This document discloses the reaction of 1,3-dimethyladamantane with formamide under acidic conditions to yield 1-formamido-3,5-dimethyladamantane.

The term "alkoxides" encompasses an alkoxide group comprising from 1 to 6 carbon atoms.

In one embodiment, the alkoxide group is methylate.

In one embodiment, said alkali metals are metals such as lithium, sodium, potassium, cesium.

In one embodiment, said earth alkali metals are metals such as calcium, barium, strontium.

In one embodiment, the protic solvent is selected from alcohols having from 1 to 8 carbon atoms, such as methanol, ethanol, n-propanol, iso-propanol, and the various isomers of butanol such as n-butanol, iso-butanol, t-butanol or s-butanol.

In another embodiment, the protic solvent is ethylene glycol or oligo(ethylene glycol). The term "oligo(ethylene glycol)" encompasses diethylene glycol, triethylene glycol, tetraethylene glycol, pentaethylene glycol, and mixtures thereof.

It has been discovered that the hydrolysis rate can be considerably accelerated if the protic solvent comprises from 10 to 60 % by weight water based on the total amount of water and solvent.

In one embodiment, the solvent is methanol that comprises from 20 to 50 % by weight water based on the total amount of water and solvent, and the base is sodium hydroxide or potassium hydroxide. Surprisingly, such a system provides particularly short hydrolysis times of approximately 1 hour resulting in favorable space-time-yields.

In one embodiment, said butanol is n-butanol.

Employing a base and a solvent that result in said short reaction times also results in high conversion rates of 1-formamido-3,5-dimethyladamantane to 1-amino-3,5-dimethyl-adamantane. In one embodiment, the crude product contains more than 95 % of the desired deacylated product.

In one embodiment, the reaction temperature is from 60 °C to 200 °C.

In other embodiments, the reaction temperature is from 60 to 180 °C, or 60 to 160 °C, or 60 to 140 °C.

In other embodiments, as reaction temperature of the hydrolysis reaction, the reflux temperature of said protic solvent may be used.

In one embodiment, the molar ratio of 1-formamido-3,5-dimethyladamantane to base may be varied within relatively broad ranges, such as 1:1 to 1:6.

In another embodiment, the amount of the protic solvent is from 10 to 50 % by weight based on the total amount of 1-formamido-3,5-dimethyladamantane, base and protic solvent.

In one embodiment, after the conversion is completed, the reaction mixture may be further diluted with water and then the desired product is separated off as the organic phase.

The conversion of 1-formamido-3,5-dimethyladamantane to 1-amino-3,5-dimethyladamantane may be monitored by gas-phase chromatography. Such methods are known to the person skilled in the art.

In one embodiment, after the hydrolysis reaction is completed, it is also possible to extract 1-amino-3,5-dimethyladamantane from the aqueous phase by means of a suitable organic solvent such as methylene chloride or toluene.

The organic phase can be dried according to the known methods, e.g. by adding anhydrous sodium sulfate or by azeotropic distillation. After filtering off sodium sulfate and evaporating the organic solvent in vacuum, the desired product is obtained.

Accordingly, in one embodiment, the method further comprises:
(ii) separating 1-amino-3,5-dimethyladamantane obtained in step (i) from the base and the protic solvent.

This separation is preferably achieved by addition of water of by distillation of the protic solvent used in the hydrolysis.

This separation preferably is partial or complete.

Subsequent addition of an acid results in the protonation of the amino group of memantine.

Accordingly, in a further embodiment, the method further comprises:
(iii) adding an acid to 1-amino-3,5-dimethyladamantane obtained in step (i) or in step (ii).

In one embodiment, hydrochloric acid is used as acid.

Hydrochloric acid may be introduced in aqueous form or as a gas (direct or pre-absorbed in a solvent).

The temperature of the acid added preferably is form 0°C to 100°C, preferably 10°C to 40°C, further preferably 20 to 30°C.

The salt of 1-amino-3,5-dimethyladamantane may be isolated either by filtering off or by removing the solvent in vacuum.

In one embodiment, the term "salt" defines a pharmaceutically acceptable salt of 1-amino-3,5-dimethyladamantane.

Pharmaceutically acceptable salts include, but are not limited to, acid addition salts, such as those made with hydrochloric, methylsulfonic, hydrobromic, hydroiodic, perchloric, sulfuric, nitric, phosphoric, acetic, propionic, glycolic, lactic, pyruvic, malonic, succinic, fumaric, tartaric, citric, benzoic, carbonic, cinnamic, mandelic, methanesulfonic, ethanesulfonic, hydroxyethanesulfonic, benzenesulfonic, p-toluene sulfonic, cyclohexanesulfamic, salicyclic, p-aminosalicylic, 2-phenoxybenzoic, and 2-acetoxybenzoic acid. The nature of the salt is not critical, provided that it is non-toxic and does not substantially interfere with the desired pharmacological activity.

The term "pharmaceutically acceptable" as used in connection with compositions of the invention, refers to molecular entities and other ingredients of such compositions that are physiologically tolerable and do not typically produce untoward reactions when administered to a mammal (e.g., human). The term "pharmaceutically acceptable" may also mean the approval by a regulatory agency of a Federal government or a state government, or the listing in the U.S. Pharmacopeia or other generally recognized pharmacopeia for use in mammals, and more particularly in humans.

In one embodiment, such a pharmaceutically acceptable salt is prepared by a method as set forth below.

The above mentioned extraction of 1-amino-3,5-dimethyladamantane from said aqueous phase as decribed in connection with methylene chloride or toluene, however, is not limited to said solvents. Organic solvents different from methylene chloride or toluene may be used.

In one embodiment, said solvent is an aprotic solvent.

The term "aprotic solvent" encompasses all solvents that do not contain a functional group from which a proton may be dissociated off.

In one embodiment, the aprotic solvent may be selected from the group consisting of aprotic polar or aprotic non-polar solvents.

In one embodiment, aprotic non-polar solvents are selected from the group consisting of (halogenated) alkanes, cycloalkanes, alkenes, aromatic hydrocarbons, in particular compounds of the benzene type such as benzene or toluene, carboxylic acid esters such acidic acid ethylester, cyclic or non-cyclic ethers such a diethyl ether or tetrahydrofurane, halogenated hydrocarbons such as methylene chloride, or mixtures of two or more of said solvents. In another embodiment, aprotic polar solvents are selected from the group consisting of ketones such as acetone, or lactones such as 4-butyrolactone, or esters.

In one embodiment, the aprotic solvent, respectively aprotic non-polar solvent, is selected from alkanes.

In one embodiment, said alkane is selected from the group consisting of pentane, hexane, heptane, octane, nonane, decane, or a mixture of two or more of said solvents.

In one embodiment, said aprotic solvent is selected from hexane, heptane, cyclohexane, toluene, dichloromethane, diethylether, MTBE, MTHF, MEK, EtOAc or iPrOAc.

Said solvents may comprise the mentioned alkane as one isomeric compound, or in the form of a mixture of isomeric compounds.

In one embodiment, said solvent is heptane. In one embodiment, n-heptane is employed as aprotic solvent.

In one embodiment, it is not necessary to isolate the formed 1-amino-3,5-dimethyladamantane as such, but to subsequently process the solution thereof in said aprotic solvent. Said solvent is preferably "carried through" most of, if not all of the further processing steps. This is advantageous in view of an industrial realization.

In one embodiment, step (ii) further comprises step (ii'):
(ii') dissolving 1-amino-3,5-dimethyladamantane in an aprotic solvent.

In one embodiment, said solution of 1-amino-3,5-dimethyladamantane in said aprotic solvent is subjected to a purification step.

In one embodiment, said purification step is performed in order to remove discoloration or odors from said solution.

In one embodiment, the solution comprising said dissolved 1-amino-3,5-dimethyladamantane is subjected to charcoal. In another embodiment, said solution is subjected to a chromatographical purification step.

Accordingly, said method further comprises step (iv):
(iv) subjecting said aprotic solvent comprising said dissolved 1-amino-3,5-dimethyladamantane to a purification step.

In another embodiment, in order to isolate the addition salt of 1-amino-3,5-dimethyladamantane with hydrochloric acid, the solvent comprising said 1-amino-3,5-dimethyladamantane is subjected to hydrogen chloride.

In one embodiment, said hydrogen chloride is added in gaseous form to said solvent comprising said 1-amino-3,5-dimethyladamantane.

In another embodiment, said hydrogen chloride is added in dissolved form to said solvent comprising said 1-amino-3,5-dimethyladamantane. In one embodiment, prior to said addition, said hydrogen chloride is dissolved in a polar solvent such as water or an alcohol, such as ethyl alcohol or propanol.

In one embodiment, said hydrogen chloride is dissolved in propanol such as isopropanol.

Accordingly, in one embodiment, the method further comprises step (v):
(v) adding hydrogen chloride to said aprotic solvent comprising said dissolved 1-amino-3,5-dimethyladamantane obtained in step (ii') or step (iv).

In general, already during the addition of hydrogen chloride either in gaseous form or in dissolved form to said aprotic solvent comprising said 1-amino-3,5-dimethyladamantane, the respective addition salt of 1-amino-3,5-dimethyladamantane with hydrogen chloride starts precipitating from the resulting mixture.

In one embodiment, after the addition salt has been precipitated, said mixture is heated in order to remove any excess of hydrogen chloride that might be present in the mixture, and/or to effect a favorable crystallisation/recrystallisation in said aprotic solvent in order to achive a particle size distribution being as narrow as possible, and/or to extract impurities from the formed addition salt.

In one embodiment, crystallisation/recrystallisation is carried out in a manner such that the chloride is obtained in particle sizes that allow direct tabletting, i.e. are obtained such that milling or sieving is not necessary.

In this respect, it is noteworthy that crystals having particle sizes of more than 500 µm may often only be directly pressed to a pharmaceutical composition such as a tablet form under difficulties, since they are relatively coarse and hard and render the tablet inhomogeneous. Prior to the pressing, such coarse and hard crystals may have to be broken by appropriate means known in the art.

In one embodiment, the instant invention pertains to 1-amino-3,5-dimethyladamantane hydrochloride in the form of crystals having a particle size distribution of d(90) in the range of from 100 µm to 500 µm, or 100 µm to 300 µm, or 150 µm to 300 µm, or 200 µm to 275 µm, or 200 µm to 250 µm as measured with laser diffraction.

In a further embodiment, the 1-amino-1,3-dimethyladamantane in the form of crystals having a particle size distribution of d(90) in the range of 200 µm to 300 µm and 10 % or less having a particle size of 55 µm or less, e.g. 5 µm or less.

The particle size range of the resulting crystals is sufficient that further milling of the crystals prior to pressing them to a tablet is not necessary. It is, however, well within the ambit of the present invention to mill or sieve the particles as obtained from the process as claimed herein.

A particle size distribution d(90) in the range of from 100 µm to 500 µm may be favorable in view of a galenical formulation and/or administration to patients.

In one embodiment, said mixture obtained in step (v) is heated to a temperature of from 30 °C up to 100 °C, or 40 °C up to 90 °C, or 50 °C up to 80 °C.

Accordingly, in one embodiment, the method further comprises step (vi):
(vi) heating the mixture obtained in step (v) to a temperature of from 50 to 75 °C.

In one embodiment, after said heating, said precipitated salt is isolated.

In one embodiment, said isolation is performed by filtration. Subsequent to the filtration, said filter cake may be washed with further solvent and may be dried, e.g. may be dried in vacuum.

In one embodiment, after said heating and prior to said filtering, said mixture is cooled down to ambient temperature, or to a temperature of from 0 °C to 10 °C.

Subsequent to said cooling down, said mixture may be processed by filtering said salt off, and washing and drying said salt.

The process according to the invention allows for obtaining 1-amino-3,5-dimethyladamantane hydrochloride in high yield, wherein said hydrochloride has a particle size distribution that is directly suitable for tabletting, i.e. without milling prior to the tabletting. This is particularly advantageous for an economical industrial process.

In one embodiment, 1-amino-3,5-dimethyladamantane hydrochloride is obtained in a high purity.

In one embodiment, the purity is between 99.0 and 99.9 %.

The new method yields the target product 1-amino-3,5-dimethyladamantane from 1-formamido-3,5-dimethyladamantane in high conversion rates, with high hydrolysis rates, i.e. short hydrolysis times, and in excellent yields. Further, due to the absence of acidic components during hydrolysis, corrosion of the equipment may be avoided. Also salts such as the pharmaceutically acceptable hydrochloride of memantine may be obtained in good yields by adding an acid to 1-amino-3,5-dimethyladamantane. Said salt formation resulting in a narrow particle size distribution and in a product of high purity. Said salt forming process may be advantageously performed in an alkane such as heptane as solvent. The combination of heating 1-formamido-3,5-dimethyladamantane with a base in a protic solvent with the subsequent step of hydrochloride formation of the produced 1-amino-3,5-dimethyladmantane in heptane using hydrogen chloride as described above allows a favorable production of 1-amino-3,5-dimethyladamantane hydrochloride both on an industrial and economical scale.

### EXAMPLES

### Example 1

**Hydrolysis of 1-formamido-3,5-dimethyladamantane** 1-formamido-3,5-dimethyladamantane (technical grade, purity 70 %, 2 mmol, calculated for 100 % pure) was heated with the selected base (6 mmol) in selected solvent (10 ml) or solvent system (10 ml) at reflux temperature respectively for ethylene glycol heating at 140 °C. The reaction was monitored by taking aliquots after the time indicated in the examples. Said aliquots were partitioned between toluene and water. The organic phase was separated and dried over anhydrous Na₂SO₄ and was analyzed by gas-phase chromatography. The conversion rate [%] of 1-formamido-3,5-dimethyladamantane to 1-amino-3,5-dimethyladamantane was calculated according to the formula: quantity of 1-formamido-3,5-dimethyladamantane * 100 %/quantity of 1-formamido-3,5-dimethyladamantane + 1-amino-3,5-dimethyladamantane.

Hydrolysis in n-butanol (1 h; reflux temperature; comparative)

| Base: | NaOH | KOH | NaOCH₃ | CsOH*H₂O |
|---|---|---|---|---|
| Conversion rate: | 100 | 98,5 | 93,5 | 100 |

Hydrolysis in n-butanol (5 h; reflux temperature; comparative):

| Base: | NaOH | KOH | NaOCH₃ | CsOH*H₂O |
|---|---|---|---|---|
| Conversion rate: | 100 | 100 | 100 | 100 |

Hydrolysis in ethylene glycol (1 h; 140 °C; comparative):

| Base: | NaOH | KOH |
|---|---|---|
| Conversion rate: | 97 | 97 |

Hydrolysis in ethylene glycol ((5 h; 140 °C; comparative):

| Base: | NaOH | KOH |
|---|---|---|
| Conversion rate: | 100 | 100 |

Hydrolysis in methanol / water (5 h; reflux temperature):

| Methanol containing | 80 % water comparative | 50 % water | 20 % water | 0,1 % water comparative |
|---|---|---|---|---|
| Conversion rate for NaOH | 62 | 99 | 90 | 63 |
| Conversion rate for KOH | 57 | 92 | - | 53 |

Hydrolysis rate in % in methanol / water (10 h; reflux temperature):

| Methanol containing | 80 % water comparative | 50 % water | 20 % water | 0,1 % water comparative |
|---|---|---|---|---|
| Conversion rate for NaOH | 83 | 100 | 99 | 81 |
| Conversion rate for KOH | 75 | 98 | - | 71 |

### Example 2 comparative

### Preparative synthesis of memantine hydrochloride from 1-formamido-3,5-dimethyladamantane:

A mixture of 1-formamido-3,5-dimethyladamantane (purity 96 % (5mmol)), base (15 mmol), solvent (25 ml) was set to reflux for the indicated time. The mixture was cooled to room temperature and diluted with water (25 to 100 ml). The product was taken into diethyl ether (25 ml). The organic phase was separated and dried over anhydrous Na₂SO₄. The solution was filtered and to this added was 2 m HCl in diethyl ether (4 ml, 8 mmol). The solvent was removed in vacuum and the residue treated with diethyl ether, filtered and dried in vacuum over phosphorus pentoxide.

| **Solvent** | **Base** | **Time [h]** | **Yield [%]** |
|---|---|---|---|
| n-butanol (comparative) | NaOH | 1 | 87 (purity 97 %) |
| n-butanol (comparative) | KOH | 1 | 85 (purity 97 %) |
| ethylene glycol (comparative) | NaOH | 5 | 74 (purity 99 %) |
| methanol / 20 % water | NaOH | 12 | 85 (purity 99 %) |
| methanol / 50 % water | KOH | 20 | 83 (purity 100%) |

### Example 3

### Hydrolysis of 1-formamido-3,5-dimethyladamantane and formation of the hydrochloride as pharmaceutically acceptable salt

1-formamido-3,5-dimethyladamantane (purity 93 %; 1.7 mol, calculated for 100 % pure), sodium hydroxide (3 molar equivalents), 350 ml water, and 740 ml methanol were heated at reflux temperature for 12 hours. After the mixture had been cooled down to approx. 35 °C, 1.000 ml of n-heptane were added and the resulting mixture was stirred for 60 minutes. The organic phase was separated off and washed three times with water. Subsequent to the washing, the organic phase was stirred with 6 g of charcoal for 30 minutes. After the charcoal had been filtered off, approx. 1.3 molar equivalents of gaseous hydrogen chloride were fed into the liquid. The resulting suspension was heated to a temperature of from 60 to 65 °C. After cooling down to approx. 0 °C, the solid was filtered off, was washed twice with n-heptane and was dried in vacuum (86 % yield, purity: 99.8%).

## Claims

1. Method for producing 1-amino-3,5-dimethyladamantane or a salt thereof, comprising:
(i) heating 1-formamido-3,5-dimethyladamantane with a base in a protic solvent;
wherein the base is selected from hydroxides, alkoxides, oxides, carbonates and hydrogen carbonates of alkali metals and earth alkali metals; and
wherein the protic solvent is selected from alcohols having from 1 to 8 carbon atoms; ethylene glycol, oligo(ethylene glycol); and
wherein the protic solvent comprises from 10 to 60 % by weight water based on the total amount of water and solvent.

2. Method of claim 1, wherein the solvent is methanol that comprises from 20 to 50 % by weight water based on the total amount of water and solvent, and the base is sodium hydroxide or potassium hydroxide, or wherein the solvent is ethylene glycol and the base is sodium hydroxide or potassium hydroxide.

3. Method of claim 1 or 2, wherein the solvent is n-butanol and the base is sodium hydroxide or potassium hydroxide or cesium hydroxide.

4. Method of any one of the preceding claims, wherein step (i) is carried out at a temperature of from 60 to 200 °C.

5. Method of any one of the preceding claims, wherein the molar ratio of 1-formamido-3,5-dimethyladamantane to base is from 1:1 to 1:6.

6. Method of any one of the preceding claims, wherein the amount of the protic solvent is from 10 to 50 % by weight based on the total amount of 1-formamido-3,5-dimethyladamantane, base and protic solvent.

7. Method of any one of the preceding claims, further comprising:
(ii) separating 1-amino-3,5-dimethyladamantane obtained in step (i) from the base and the protic solvent.

8. Method of any one of the preceding claims, further comprising:
(iii) adding an acid to 1-amino-3,5-dimethyladamantane obtained in step (i) or in step (ii).

9. Method of claim 8, wherein said acid is hydrochloric acid.

10. Method of any one of claims 7 to 9, wherein step (ii) comprises step (ii'):
(ii') dissolving 1-amino-3,5-dimethyladamantane in an aprotic solvent.

11. Method of claim 10, wherein said aprotic solvent is an alkane.

12. Method of claim 11, wherein said alkane is selected from the group consisting of pentane, hexane, heptane, octane, nonane, decane, or a mixture of two or more of said solvents.

13. Method of claim 12, wherein said alkane is heptane.

14. Method of any one of claims 10 to 12, further comprising step (iv):
(iv) subjecting said aprotic solvent comprising said dissolved 1-amino-3,5-dimethyladamantane obtained in step (ii') to a purification step.

15. Method of any one of claims 10 to 14, further comprising at least one of the following steps:
(v) adding hydrogen chloride to said aprotic solvent comprising said dissolved 1-amino-3,5-dimethyladamantane obtained in step (ii') or step (iv) or
(vi) heating the mixture obtained in step (v) to a temperature of from 50 to 75 °C.

## Patentansprüche

1. Verfahren zur Herstellung von 1-Amino-3,5-dimethyladamantan oder eines Salzes davon, umfassend:
(i) Erhitzen von 1-Formamido-3,5-dimethyladamantan mit einer Base in einem protischen Lösungsmittel;
wobei die Base ausgewählt ist aus Hydroxiden, Alkoxiden, Oxiden, Carbonaten und Hydrogencarbonaten von Alkalimetallen und Erdalkalimetallen; und
wobei das protische Lösungsmittel ausgewählt ist aus Alkoholen mit 1 bis 8 Kohlenstoffatomen; Ethylenglykol, Oligoethylenglykol; und
wobei das protische Lösungsmittel 10 bis 60 Gew.-% Wasser umfasst, bezogen auf die Gesamtmenge an Wasser und Lösungsmittel.

2. Verfahren nach Anspruch 1, wobei das Lösungsmittel Methanol ist, welches 20 bis 50 Gew.-% Wasser umfasst, bezogen auf die Gesamtmenge an Wasser und Lösungsmittel, und wobei die Base Natriumhydroxid oder Kaliumhydroxid ist, oder wobei das Lösungsmittel Ethylenglykol und die Base Natriumhydroxid oder Kaliumhydroxid ist.

3. Verfahren nach Anspruch 1 oder 2, wobei das Lösungsmittel n-Butanol und die Base Natriumhydoxid oder Kaliumhydroxid oder Cäsiumhydroxid ist.

4. Verfahren nach einem der vorstehenden Ansprüche, wobei Stufe (i) bei einer Temperatur von 60 bis 200 °C durchgeführt wird.

5. Verfahren nach einem der vorstehenden Ansprüche, wobei das molare Verhältnis von 1-Formamido-3,5-dimethyladamantan zur Base 1:1 bis 1:6 ist.

6. Verfahren nach einem der vorstehenden Ansprüche, wobei die Menge an protischem Lösungsmittel 10 bis 50 Gew.-% beträgt, bezogen auf die Gesamtmenge an 1-Formamido-3,5-dimethyladamantan, Base und protischem Lösungsmittel.

7. Verfahren nach einem der vorstehenden Ansprüche, weiter umfassend:
(ii) Abtrennen von 1-Amino-3,5-dimethyladamantan, welches in Stufe (i) erhalten wurde, von der Base und dem protischen Lösungsmittel.

8. Verfahren nach einem der vorstehenden Ansprüche, weiter umfassend:
(iii) Hinzufügen einer Säure zum 1-Amino-3,5-dimethyladamantan, welches in Stufe (i) oder in Stufe (ii) erhalten wurde.

9. Verfahren nach Anspruch 8, wobei besagte Säure Salzsäure ist.

10. Verfahren nach einem der Ansprüche 7 bis 9, wobei Stufe (ii) die Stufe (ii') umfasst:
(ii') Auflösen von 1-Amino-3,5-dimethyladamantan in einem aprotischen Lösungsmittel.

11. Verfahren nach Anspruch 10, wobei besagtes aprotisches Lösungsmittel ein Alkan ist.

12. Verfahren nach Anspruch 11, wobei besagtes Alkan ausgewählt ist aus der Gruppe bestehend aus Pentan, Hexan, Heptan, Oktan, Nonan, Dekan oder einer Mischung aus zwei oder mehreren dieser Lösungsmittel.

13. Verfahren nach Anspruch 12, wobei besagtes Alkan Heptan ist.

14. Verfahren nach einem der Ansprüche 10 bis 12, weiter umfassend Stufe (iv):
(iv) Unterziehen des besagten aprotischen Lösungsmittels, welches besagtes gelöste 1-Amino-3,5-dimethyladamantan umfasst, welches in Stufe (ii') erhalten wurde, einem Reinigungsschritt.

15. Verfahren nach einem der Ansprüche 10 bis 14, weiter umfassend mindestens eine der folgenden Stufen:
(v) Hinzufügen von Chlorwasserstoff zum besagten aprotischen Lösungsmittel, welches besagtes gelöste 1-Amino-3,5-dimethyladamantan umfasst, welches in Stufe (ii') oder Stufe (iv) erhalten wurde, oder
(vi) Erhitzen der Mischung, weiche in Stufe (v) erhalten wurde, auf eine Temperatur von 50-75 °C.

## Revendications

1. Procédé de production de 1-amino-3,5-diméthyladamantane ou d'un sel de celui-ci, comprenant :
(i) le chauffage de 1-formamido-3,5-diméthyladamantane avec une base dans un solvant protique ;
dans lequel la base est choisie parmi des hydroxydes, des alcoxydes, des oxydes, des carbonates et des hydrogénocarbonates de métaux alcalins et de métaux alcalinoterreux ; et
dans lequel le solvant protique est choisi parmi des alcools ayant de 1 à 8 atomes de carbone ; l'éthylèneglycol, un oligo(éthylèneglycol) ; et
dans lequel le solvant protique comprend de 10 à 60 % en masse d'eau rapporté à la quantité totale d'eau et de solvant.

2. Procédé selon la revendication 1, dans lequel le solvant est du méthanol qui comprend de 20 à 50 % en masse d'eau rapporté à la quantité totale d'eau et de solvant, et la base est de l'hydroxyde de sodium ou de l'hydroxyde de potassium, ou dans lequel le solvant est de l'éthylèneglycol et la base est de l'hydroxyde de sodium ou de l'hydroxyde de potassium.

3. Procédé selon la revendication 1 ou 2, dans lequel le solvant est le n-butanol et la base est l'hydroxyde de sodium ou l'hydroxyde de potassium ou l'hydroxyde de césium.

4. Procédé selon l'une quelconque des revendications précédentes, dans lequel l'étape (i) est réalisée à une température de 60 à 200°C.

5. Procédé selon l'une quelconque des revendications précédentes, dans lequel le rapport molaire du 1-formamido-3,5-diméthyladamantane à la base est de 1:1 à 1:6.

6. Procédé selon l'une quelconque des revendications précédentes, dans lequel la quantité du solvant protique est de 10 à 50 % en masse rapporté à la quantité totale de 1-formamido-3,5-diméthyladamantane, de base et de solvant protique.

7. Procédé selon l'une quelconque des revendications précédentes, comprenant de plus :
(ii) la séparation de 1-amino-3,5-diméthyladamantane obtenu dans l'étape (i) de la base et du solvant protique.

8. Procédé selon l'une quelconque des revendications précédentes, comprenant de plus :
(iii) l'addition d'un acide au 1-amino-3,5-diméthyladamantane obtenu dans l'étape (i) ou dans l'étape (ii).

9. Procédé selon la revendication 8, dans lequel ledit acide est l'acide chlorhydrique.

10. Procédé selon l'une quelconque des revendications 7 à 9, dans lequel l'étape (ii) comprend l'étape (ii') :
(ii') de dissolution de 1-amino-3,5-diméthyladamantane dans un solvant aprotique.

11. Procédé selon la revendication 10, dans lequel ledit solvant aprotique est un alcane.

12. Procédé selon la revendication 11, dans lequel ledit alcane est choisi dans le groupe constitué de pentane, d'hexane, d'heptane, d'octane, de nonane, de décane, ou d'un mélange de deux ou plusieurs desdits solvants.

13. Procédé selon la revendication 12, dans lequel ledit alcane est l'heptane.

14. Procédé selon l'une quelconque des revendications 10 à 12, comprenant de plus l'étape (iv) :
(iv) de soumission dudit solvant aprotique comprenant ledit 1-amino-3,5-diméthyladamantane dissous obtenu dans l'étape (ii') à une étape de purification.

15. Procédé selon l'une quelconque des revendications 10 à 14, comprenant de plus au moins une des étapes suivantes :
(v) d'addition de chlorure d'hydrogène audit solvant aprotique comprenant ledit 1-amino-3,5-diméthyladamantane dissous obtenu dans l'étape (ii') ou l'étape (iv) ou
(vi) de chauffage du mélange obtenu dans l'étape (v) à une température de 50 à 75°C.
